# EUROPEAN PATENT SPECIFICATION

(11) **EP 3 060 149 B1**
(45) Date of publication and mention of the grant of the patent: **31.01.2018**
(21) Application number: 14810042.3
(22) Date of filing: 18.10.2014
(51) Int. Cl.: A61B 18/02

(54) **TROCAR WITH A FREEZING PROBE**
TROKAR MIT EINER GEFRIERSONDE
TROCART À SONDE CRYOGÉNIQUE

(30) Priority: 18.10.2013 PL 40568313; 04.04.2014 IT MI20140614
(43) Date of publication of application: 31.08.2016
(73) Proprietor: Warszawski Uniwersytet Medyczny, 02-091 Warszawa (PL)
(72) Inventor: STRULAK, Lukasz, 04-566 Warszawa (PL)
(74) Representative: Witek, Rafal
(86) International application number: PCT/PL2014/050066
(87) International publication number: WO 2015/057092

(56) References cited:
- US-A1- 2001 037 081
- US-A1- 2005 043 724
- US-A1- 2012 089 047

## Description

The subject of the present invention is a sharp/blunt trocar with a freezing probe for freezing tissues suitable for freezing tissues, in particular the sympathetic plexus leading to the kidneys and renal artery used in the regulation of arterial pressure.
Patent US 3298371 discloses a freezing probe used particularly in neurosurgery. The probe consists of ducts for conducting a freezing agent, i.e. liquid nitrogen, terminating in a heat-conducting head. The head contains a detector, which measures its temperature in real time in order to maintain appropriate conditions for the procedure. The aforementioned probe possesses a head with a diameter of about 0.09 inch, wherein it is not deformed elastically and thus is not useful in the effective freezing of tissues on a large surface such as the sympathetic plexus.
Patent US6306129 discloses in turn a system for cardiosurgery containing a compressor and a probe. The probe has a secondary cooling channel, which performs an initial cooling at a resting state. After the initial cooling, the main cooling stream is initiated, which facilitates temperatures near - 100°C. This probe does not possess an elastic head and therefore the active freezing surface is limited only to the tissue-head contact point. Large surface activity is thus difficult, particularly that of a cylindrical shape.
Patent US7063718 discloses a method and apparatus for performing a hypothermy of a selected organ without a significant effect on adjacent organs and tissues. The elastic catheter is inserted through the patient's vasculature in order to insert the end of the freezing probe through the artery that supplies the selected organ. The compressed refrigerant is pumped in through a catheter into the decompressing element found near the end of the probe, wherein the refrigerant decompresses and evaporates which cools the elastic thermoconductive element in the probe's end. This leads to the cooling of blood flowing through a selected vessel, which then cools the selected organ. In one embodiment of said invention, the element responsible for heat exchange consists of a plurality of concentric tubes leading to the return duct for the refrigerant. The nitinol tubes remain rectilinear at room temperature, whereas they change shape at low temperatures, leading to the formation of an elastic "basket", which allows for the easier insertion of the catheter system into the patient's bloodstream with substantially rectilinear but flexible tubes. After insertion into the desired site, the refrigerant flow is initiated which causes a change in the geometry of the nitinol tubes and forms the basket thereby increasing the effective length of the refrigerant decompression path and by the same token decreasing the catheter length required to achieve the desired temperature. Organ or tissue cooling occurs through the end of the probe, which is not elastic and facilitates cooling solely at the contact interface. It is thus not possible to simultaneously cool a larger surface, i.e. in cylindrical form.

Patent application US2008071337 discloses a catheter capable of being placed for heating or cooling the surrounding fluid in a patients blood vessel. The catheter possesses a heat exchange element that contains a plurality of external surface irregularities in order to induce turbulence in the surrounding fluid. The catheter that supplies the refrigerant, the heat exchange cooling element, is equipped with balloons at its end, supplied by a separate duct. After inserting into an appropriate site, the working fluid supply catheter is withdrawn in order to provide a seal using the balloon. After sealing, the chamber is filled with the working fluid which cools the heat exchange element. The balloon solely has a sealing function, and thus does not provide any thermal activity. Moreover, the presented catheter provides solely fluid cooling and is thus not useful for the point cooling of organs or tissues. Moreover, the activity surface area is constant, not elastic, due to which there is no possibility to enclose a selected blood vessel in order to obtain better cooling.

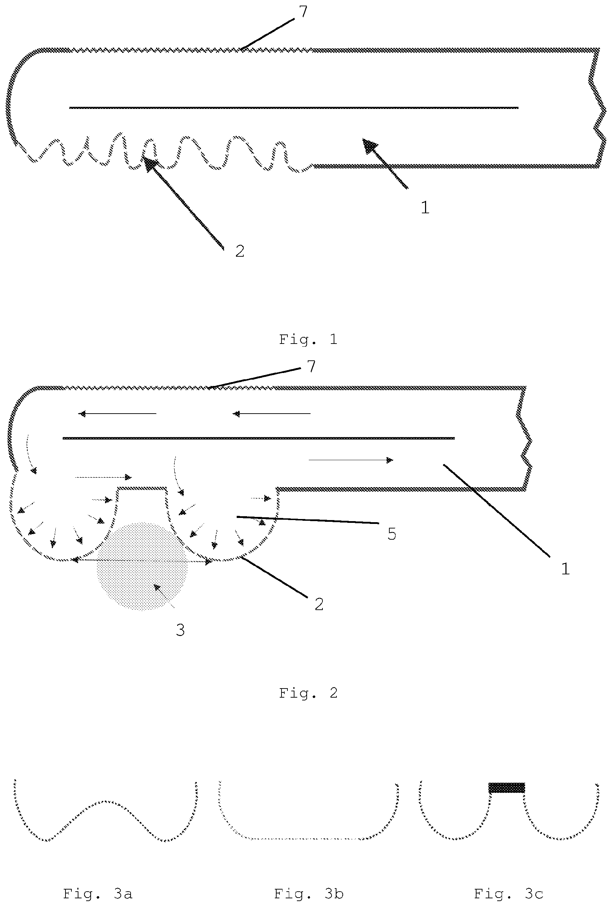

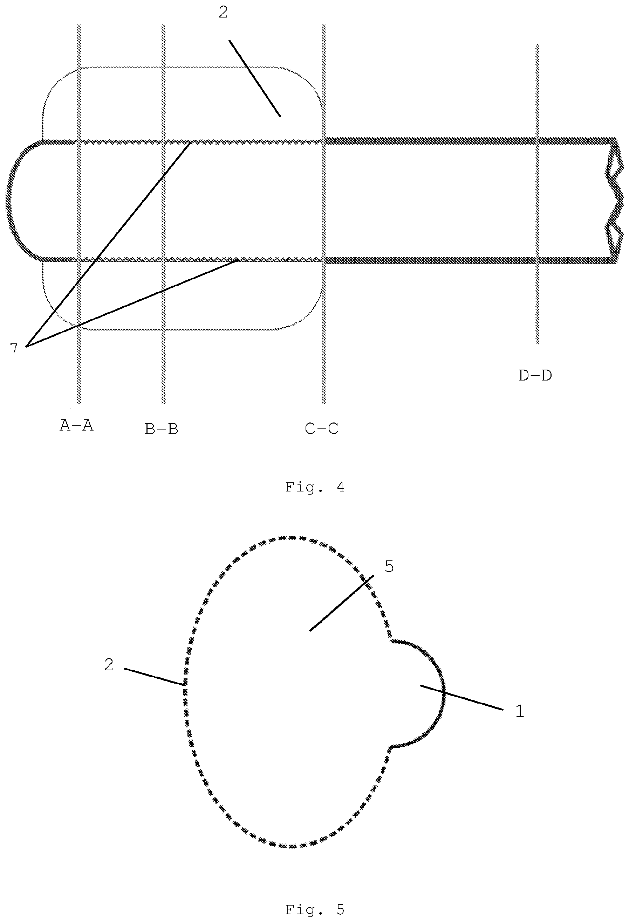

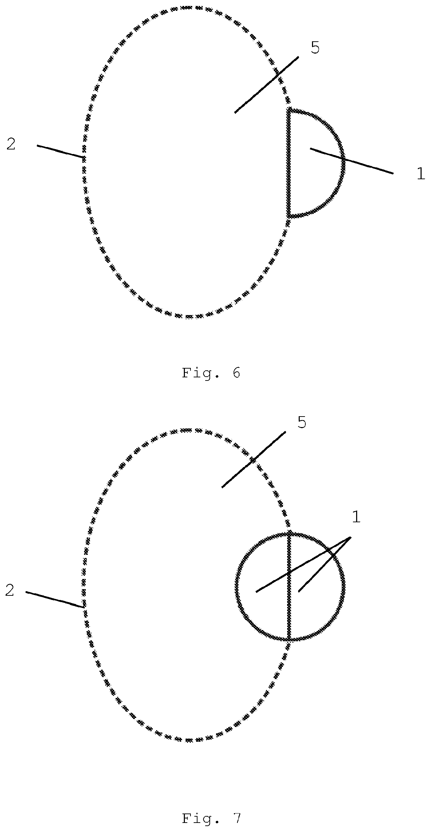

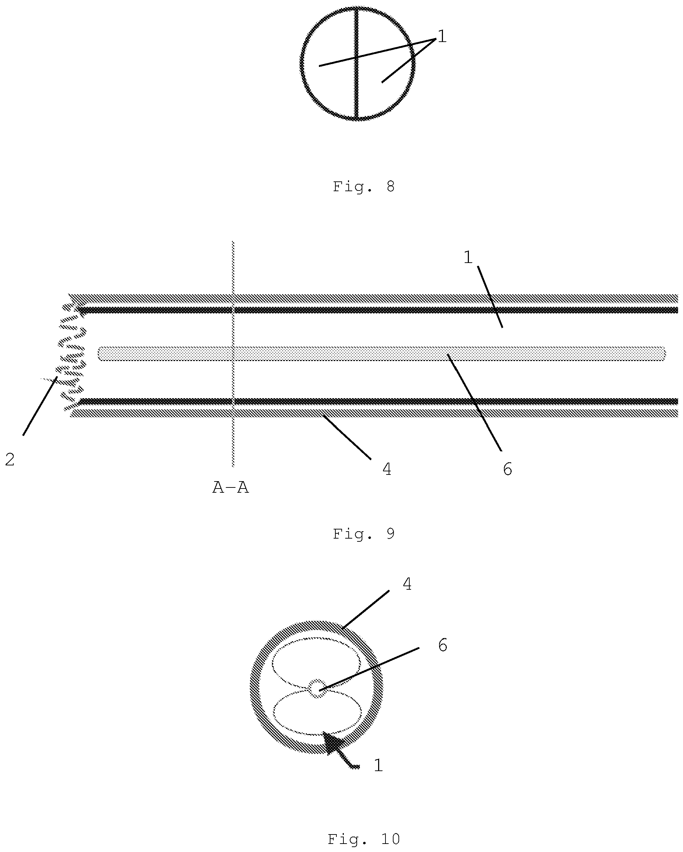

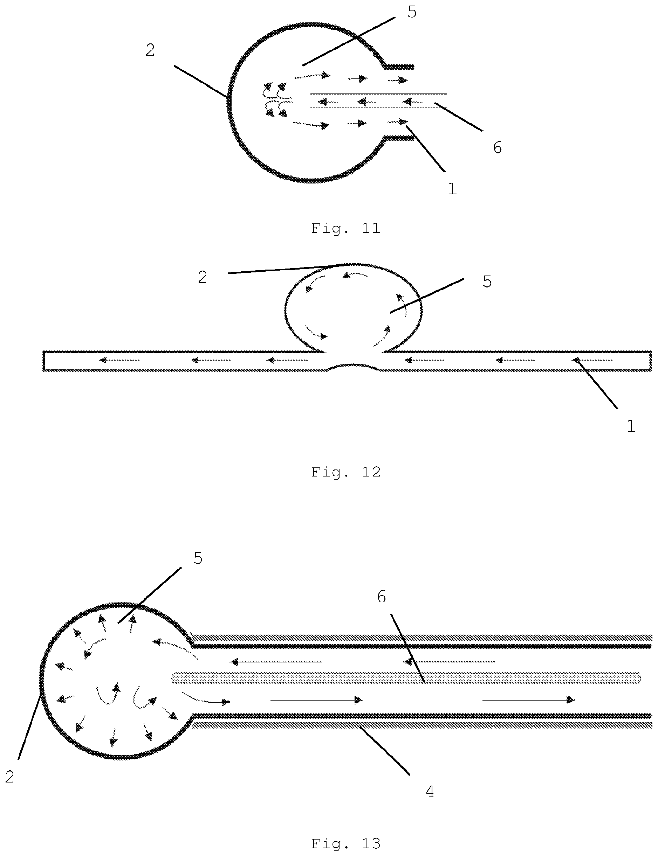

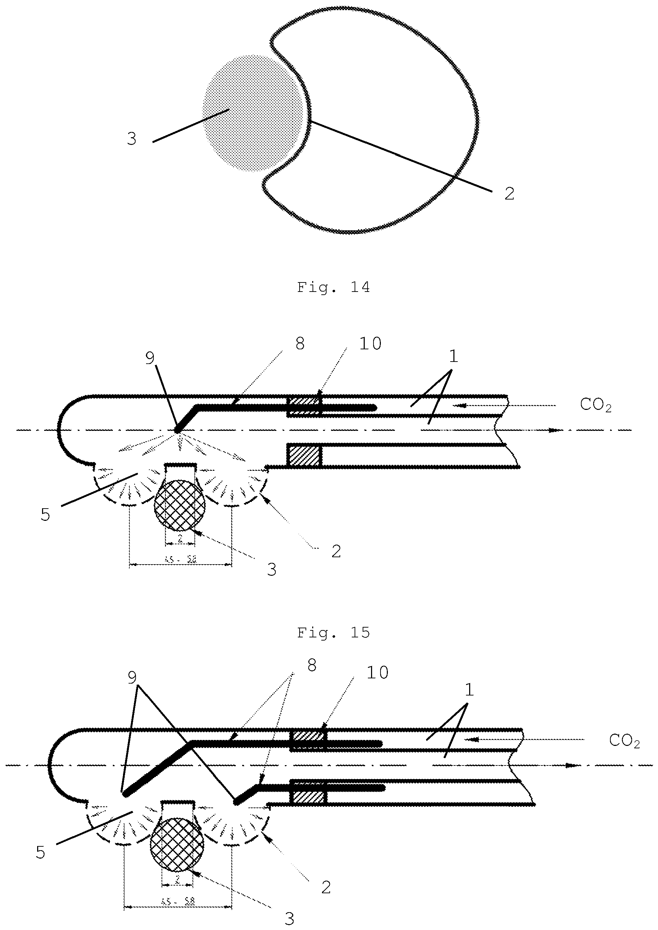

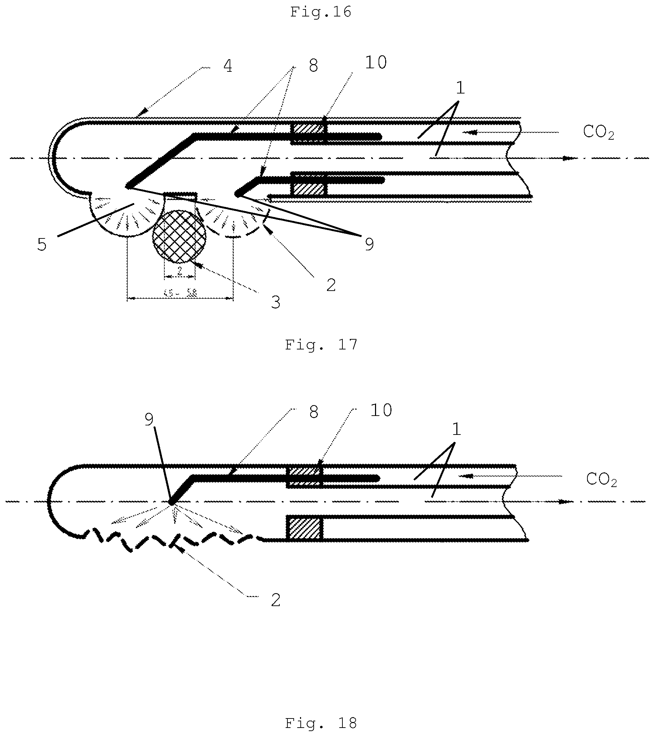

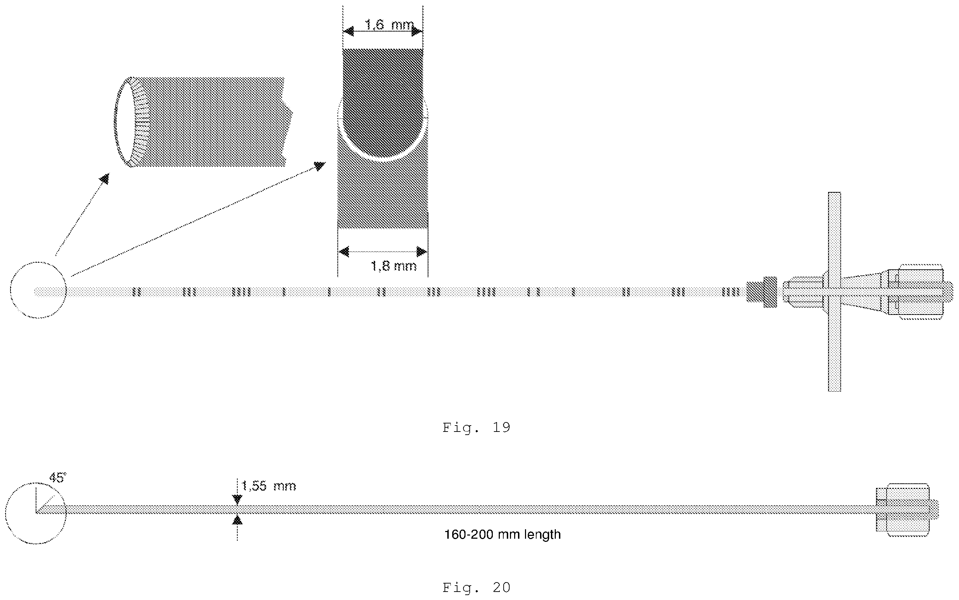

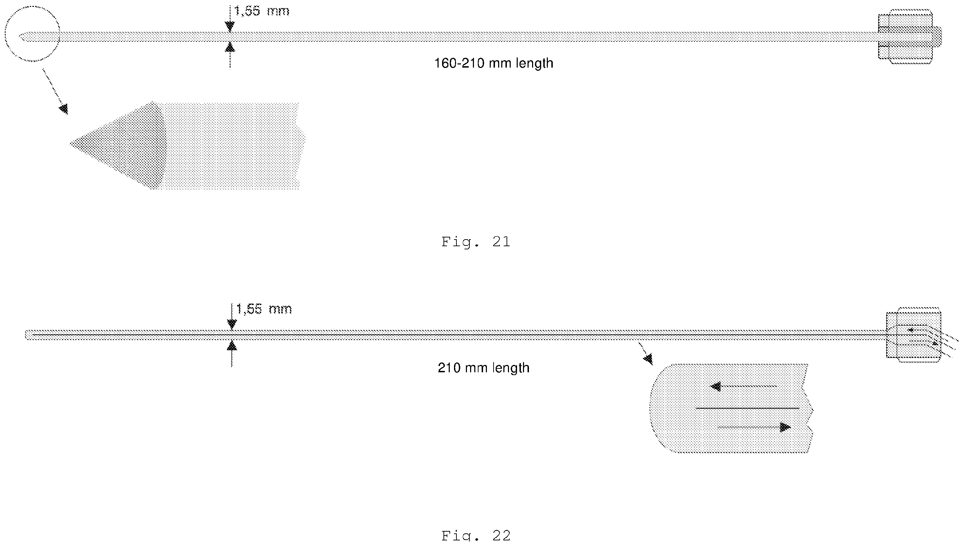

Furthermore, reference is made to the document US2001/0037081 A1. The technical problem set forth before the present invention is to disclose such a sharp/blunt trocar so as to facilitate access to the renal artery and its connected sympathetic plexus, one which would facilitate the point freezing of the probed nerve, ganglion, tissue or blood vessel, wherein the achieved temperature would be lower than -50°C, and the probe active cooling surface area interface with the treated tissue is large, facilitating the simultaneous freezing of a selected blood vessel over a large portion of its circumference using a lower quantity of refrigerant, and the trocar has a simple construction and is inexpensive to produce, making it possible to deliver single-use, sterile devices of this type, while at the same time the freezing procedure will be faster, reducing the risk of postoperative complications. Unexpectedly, the aforementioned problems have been solved by the present invention defined in the appended set of claims. The first subject of the present invention is a sharp/blunt trocar with a freezing probe for freezing tissues containing sealed channels for conducting a continuous flow of refrigerant, connected to a pump and a container for the refrigerant, terminating in a freezing head, characterised in that the freezing head possesses at least one elastic surface which changes shape and distends as a result of the flow of gas therein that is pumped under pressure, forming a decompression chamber, in which the refrigerant introduced via the channels 1 undergoes decompression and evaporation causing the surface of the elastic freezing head to cool, a the aforementioned elastic surface is capable of fitting itself to the tissue subjected to freezing. Preferably, the elastic surface occurs on the side wall of the freezing head. Equally preferably, the elastic surface occurs on the front wall of freezing head. In another preferable embodiment of the present invention the elastic surface comprises a material selected from among for example PEBA, graphene, PET or other elastic polymers. In the next preferable embodiment of the present invention, the surface of the trocar, outside of the elastic surface, is covered by a thermoinsulating material. Preferably, the insulating material comprises an aerogel. In another preferable embodiment of the present invention sharp/blunt trocar further comprises at least one outflow canal sealed via sealing portion to the channel, having at the end a nozzle which increases efficacy and targeting of the elastic surface. The aforementioned trocar may be used in the lowering of arterial blood pressure in the renal artery by freezing the nerve and/or plexus, which supplies the renal artery, through sympathetic innervation, halting the nerve's activity and halting the pressure regulatory mechanism.
A sharp/blunt trocar according to the present invention makes it possible to introduce it into a desired area and to freeze tissues locally. Due to its elastic surface, which adapts itself to the treated tissue, the freezing surface is enlarged and it is thus possible to freeze a large tissue surface in a single treatment step. The elastic surface makes it possible to partially surround the treated tissue, i.e. artery, increasing treatment effectiveness and shortening treatment times. The use of materials that conduct thermal energy well decreases refrigerant requirements, due to lowered thermal loss. The use of a sharp/blunt trocar according to the present invention makes it possible to perform an arterial blood pressure reduction in the renal artery by freezing the nerve and halting the pressure regulatory mechanism without a surgical incision (not longer than 1 cm) in the skin, over a shorter time, decreasing the risk of postoperative complications.
Example embodiments of the present invention are shown in the illustration, in which Fig. 1 shows a longitudinal section of the freezing probe of the sharp/blunt trocar with the elastic surface on the side of the freezing head, Fig. 2 shows a cross-section of the freezing probe of the trocar of Fig. 1 with two elastic surfaces during the treatment, Fig. 3. a, b, c shows consecutive configurations of the elastic surface, Fig. 4 shows the probe of a sharp/blunt trocar in top view, Fig. 5 shows a cross-section of the probe of a trocar of Fig. 4 along surface A-A, Fig. 6 shows a cross-section of the probe of a trocar of Fig. 4 along surface B-B, Fig. 5 shows a cross-section of the probe of a trocar of Fig. 7 along surface C-C, Fig. 8 shows a cross-section of the probe of a trocar of Fig. 4 along surface D-D, Fig. 9 shows a longitudinal section of a probe of the sharp/blunt trocar with the elastic surface on the front wall of the freezing head, Fig. 10 shows a cross-section of the probe of a trocar of Fig. 9 along surface C-C, Fig. 11 shows a magnification of the freezing head of a trocar of Fig. 9 during the unfolding of the elastic surface, Fig. 12 shows an unfolded, single-use probe of the freezing trocar of Fig. 9, Fig. 13 shows longitudinal section of the trocar of Fig. 9 in an embodiment with a spherical elastic surface, Fig. 14 shows the elastic surface of the freezing head of a trocar of Fig. 9 during treatment, Fig. 15 shows another embodiment of the freezing head of the trocar of Fig. 2, Fig. 16 shows another embodiment of the freezing head of a trocar of Fig. 2, Fig. 17 shows another embodiment of the freezing head of a trocar of Fig. 2, covered by thermoinsulating material, Fig. 18 shows another embodiment of the freezing head of a trocar of Fig. 1, Fig. 19 shows the trocar with the probe of the present invention, Fig. 20 shows a needle with a sharp tip enabling passage through skin after insertion through trocar, Fig. 21 shows a pencil-point needle enabling safe passage through muscle and fat tissue after insertion through trocar, Fig. 22 shows the freezing probe of the present invention.

### Example 1

Fig. 1 shows a sharp/blunt with an internally placed freezing probe. The freezing probe possesses an elastic surface 2 on the side of its head. The probe also contains sealed channels 1 that supply refrigerant to the elastic surface 2 of the freezing head. At the opposite side of the elastic surface 2 there is an identification area 7, which facilitates better visibility and localization during diagnosis using image-guided procedures with the help of medical imaging like computed tomography, magnetic resonance imaging or ultrasonography. Fig. 3a shows an embodiment of the elastic surface 2 of the freezing probe of a shape reminiscent of pincers, ensuring a larger contact surface at the tissue circumference 3 with a cylindrical cross-section. Fig. 3b shows the next embodiment of the elastic surface 2 of a freezing probe with a flat surface for universal use. Fig. 3c shows an embodiment of the elastic surface 2 of a freezing probe with two separate areas, which makes it possible to freeze tissue 3 from two sides simultaneously. Fig. 2 shows a sharp/blunt trocar with a freezing probe o elastic surface 2 placed inside with a shape as in Fig. 3c. After placing the trocar in an appropriate site of the body (the identification area 7 makes it possible to verify the location of the trocar), an expanding gas is introduced through the channels 1 that forms the elastic surface 2, in order to "inflate" the elastic surface 2 and to form the decompression chamber 5. The pumping is performed using a neutral gas, i.e. CO2. All other neutral gasses can be used to pump up the decompression chamber 5. After inflation, the refrigerant is introduced under pressure, i.e. liquid nitrogen, which in the formed decompression chamber 5 is decompressed and evaporates causing a drastic drop in the temperature inside the chambers 5. The elastic surface 2 is made of a durable elastic material with an excellent thermal conductivity, such as graphene, resulting in decreased thermal losses, and thinner elastic surfaces 2 may be used, thereby decreasing the amount of refrigerant necessary to perform the treatment. Fig. 4 shows a sharp/blunt trocar with a freezing probe with an elastic surface 2 shaped as in Fig. 3b in top view. Figs. 5, 6, 7, 8 show transverse sections through surfaces A-A, B-B, C-C, and D-D respectively as shown in Fig. 4. The above sections show that the elastic surface 2, that is used for freezing tissue 3, is located only on a particular, defined length, through which it is possible to manufacture a sharp/blunt trocar dedicated to particular medical uses. The trocar used makes it possible to freeze tissue 3 in a single operation, due to the elastic freezing surface 2, which has a desirable effect on the treatment time and risk of complications. Due to the use of an elastic surface 2 of a material with excellent thermal conductivity, it was possible to use a smaller quantity of refrigerant. The inflated elastic surface 2 formed a decompression chamber 5, which possessed a relatively large volume, achieving a low temperature, appropriate for the tissue freezing treatment, using a lower quantity of refrigerant, without the use of complicated heat exchangers.

### Example 2

Fig. 9 shows the next embodiment of a sharp/blunt trocar with a freezing probe for freezing tissues 3. In contrast to the trocar of Example 1, the elastic surface 2 is found at the front of the freezing head. Inside the probe, between the channels 1 supplying the refrigerant there is a pipe 6, which conducts the pumping gas to the elastic surface 2 and forms decompression chamber 5. This gas, as in Example 1, may be CO2. Fig. 10 shows a transverse cross-section along surface A-A of the sharp/blunt trocar with a freezing probe of Fig. 9. Fig. 11 shows the elastic surface 2 pumping process, forming a decompression chamber 5 with a neutral gas introduced using pipe 6. Fig. 12 shows an unfolded freezing probe placed inside the trocar, which discloses the simple construction of the freezing probe, enabling its production in the form of single-use, sterile freezing probes for a dedicated use. Fig. 13 shows the trocar with a freezing probe of Fig. 9 during refrigerant flow, such as liquid nitrogen. The refrigerant undergoes decompression and evaporation in the formed decompression chamber 5 causing a rapid temperature drop. The trocar with a freezing probe shown possesses on its peripheral surface a channel 1 that gives access to a pipe 4 with excellent thermal insulation, which safeguards tissues against undesirable freezing. This pipe may be composed of an aerogel for example. Fig. 14 shows a trocar with a freezing probe as per Fig. 9 during the freezing treatment of tissues 3. In this case, the tissue 3 being frozen is the renal artery. As may be seen, the shape of the elastic surface adapts to the treated tissue, yielding a large interaction surface facilitating effective freezing.

### Example 3

Another embodiment of blunt/sharp probe is illustrated in the Fig. 15. This probe is similar to that presented in Fig. 2. In contrast to the trocar of Fig. 2, Fig. 15 shows a blunt/sharp probe with two channels 1 enabling the inflow and outflow of a gas and/or refrigerant. The freezing probe possesses an elastic or expandable surface 2 on one side of its head. The probe also contains outflow canal 8 sealed via sealing portion 10 to the inflow channel 1 that supply gas and/or refrigerant to the elastic surface 2 of the freezing head. A nozzle 9 is located at the end of an outflow canal 8 increasing efficacy and targeting of the elastic or expandable surfaces 2. Between portions of elastic surface 2 lies the possible location of a target 3 aimed at freezing, which can be the tissue. Presented embodiment of the above-mentioned blunt/sharp probe operates on the same principle as the probe of Example 1. Fig. 16 presents another embodiment of blunt/sharp probe similar to that illustrated in Fig. 15, with three channels 1 enabling the inflow and outflow of a freezing gas and/or refrigerant. The freezing probe possesses an elastic or expandable surface 2 on one side of its head. The probe also contains two outflow canals 8 sealed via sealing portion 10 to the inflow channels 1 that supply gas and/or refrigerant to the elastic surface 2 of the freezing head. Two nozzles 9 are located at the end of an outflow canals 8 increasing efficacy and targeting of the elastic or expandable surfaces 2. Between portions of elastic surface 2 lies the possible location of a target 3 aimed at freezing, which can be the tissue. Presented embodiment of the above-mentioned blunt/sharp probe operates on the same principle as the probe of Example 1. Fig. 17 presents another embodiment of blunt/sharp probe similar to that illustrated in Fig. 15 and Fig. 16, with three channels 1 enabling the inflow and outflow of a freezing gas and/or refrigerant. The probe is enclosed/covered with an insulating material 4 enabling better visualization under an image guided (computed tomography/ ultrasound) procedure and/or decreasing the risk of collateral damage. The freezing probe possesses an elastic or expandable surface 2 on one side of its head. The probe also contains outflow channels sealed to the inflow channels 5 that supply refrigerant to the elastic surface 3 of the freezing head. two outflow canals 8 sealed via sealing portion 10 to the inflow channels 1 that supply gas and/or refrigerant to the elastic surface 2 of the freezing head. Two nozzles 9 are located at the end of outflow canals 8 increasing efficacy and targeting of the elastic or expandable surfaces 2. Between portions of elastic surface 2 lies the possible location of a target 3 aimed at freezing, which can be the tissue. Presented embodiment of the above-mentioned blunt/sharp probe operates on the same principle as the probe of Example 1. Fig. 18 presents another embodiment of blunt/sharp probe similar to that illustrated in Fig. 15, with two channels 1 in enabling the inflow and outflow of a freezing gas and/or refrigerant. The freezing probe possesses an elastic or expandable surface 2 on one side of its head. The probe also contains outflow canal 8 sealed via sealing portion 10 to the inflow channel 1 that supply gas and/or refrigerant to the elastic or folded/expandable surface 2 of the freezing head. A nozzle 9 is located at the end of an outflow canal 8 increasing efficacy and targeting of the elastic or expandable surfaces 2. Different embodiments of blunt/sharp probe presented in the present Example 3 can possess different configurations of the elastic surface 2 including, but not limited to, presented in Figs. 3 a-c.

### Example 4

Figures 19-22 show the whole trocar including freezing probe of Example 1. Figure 19 presents the trocar, which could be used in an intervention to guide the probe through tissue decreasing the risk of infection. It possesses marks for judgment of depth of tissue penetration as well as an isolating layer 4 and a standard luer lock root. The tip is cut in a safe manner to adjust to the inserted probe or needle decreasing risk of damage to tissue while passing through it. Figure 20 presents a needle with a sharp tip enabling passage through skin after insertion through trocar. Figure 21 presents a pencil-point needle enabling safe passage through muscle and fat tissue decreasing risk of tissue laceration of e.g. vessels. Figure 22 presents a simple freezing probe, for example from Example 1. With a freezing tip and an inflow and outflow channels 1 for the freezing gas. In figures a typical dimensions of trocar and probe are also presented.

### Example 5

We used the trocar of Example 1 for the regulation of arterial hypertension. The trocar facilitated simple access to the renal artery 3 and to the sympathetic plexus leading to the kidneys and artery, which regulates arterial pressure. Access to the renal artery 3 did not require any surgical incision in the skin (no incision longer than 1 cm). The access was through the patients back where the probe was inserted transdermally, inserting paravertebrally at L1/L2 through the soft tissues and accessing the area of the renal artery 3. The procedure was performed using a sharp/blunt trocar equipped with a freezing probe. The inhibition of the sympathetic plexus was realised through freezing the nerve or plexus, which supplies the renal artery, through sympathetic innervation, to a temperature below -50°C, wherein the freezing caused the nerve to deactivate and thus to shut down the pressure upregulating system (Renin-Angiotensin-Aldosterone axis).

## Claims

1. Sharp/blunt trocar with a freezing probe for freezing tissues containing sealed channels for conducting refrigerant in a continuous or algorithmic interrupted flow, the freezing probe being connected with a pump and a container for the refrigerant and terminating in a freezing head having at least one elastic surface (2) configured to change its shape and distend as a result of the flow of gas pumped under pressure through the trocar so as to form a decompression chamber (5), into which the refrigerant is supplied through the channels (1) and then undergoes decompression and evaporation causing the elastic surface (2) of the freezing head to cool, wherein the at least one elastic surface (2) is configured to fit to the tissue exposed to freezing (3) and partially surround the treated tissue, and wherein the at least one elastic surface (2) is arranged on the side wall of freezing head.

2. Sharp/blunt trocar according to Claim 1, wherein the at least one elastic surface (2) comprises a material selected from PEBA, graphene, PET or polymers.

3. Sharp/blunt trocar according to Claim 1 or 2, wherein the surface of the trocar, except for the at least one elastic surface (2), is covered by a thermally insulating material (4).

4. Sharp/blunt trocar according to Claim 3, wherein the thermally insulating material comprises an aerogel.

5. Sharp/blunt trocar according to any of Claims from 1 to 4, further comprising at least one outflow canal (8) sealed via sealing portion (10) to at least one of the channels (1) for conducting refrigerant and having at its distal end a nozzle (9) configured to increase efficacy and targeting of the elastic surface (2).

## Patentansprüche

1. Scharfer/stumpfer Trokar mit einer Gefriersonde zum Gefrierenlassen von Geweben, die abgedichtete Kanäle zum Durchleiten von Kühlmittel in einem kontinuierlichen oder algorithmisch unterbrochenen Strom enthält, wobei die Gefriersonde mit einer Pumpe und einem Behälter für das Kühlmittel verbunden ist und in einem Gefrierkopf endet, der wenigstens eine elastische Fläche (2) aufweist, die so konfiguriert Ist, dass sie infolge des Strömens von Gas, das unter Druck durch den Trokar gepumpt wird, ihre Form ändert und sich ausdehnt, so dass eine Dekompressionskammer (5) entsteht, in die das Kühlmittel durch die Kanäle (1) zugeführt wird und dann eine Dekompression und Verdunstung erfährt, was bewirkt, dass sich die elastische Fläche (2) des Gefrierkopfs abkühlt, wobei die wenigstens eine elastische Fläche (2) so konfiguriert ist, dass sie zu dem Gewebe, das gefrieren soll (3), passt und das behandelte Gewebe teilweise umgibt, und wobei die wenigstens eine elastische Fläche (2) auf der Seitenwand des Gefrierkopfs angeordnet ist.

2. Scharfer/stumpfer Trokar gemäß Anspruch 1, wobei die wenigstens eine elastische Fläche (2) ein Material umfasst, das aus PEBA, Graphen, PET oder Polymeren ausgewählt ist.

3. Scharfer/stumpfer Trokar gemäß Anspruch 1 oder 2, wobei die Fläche des Trokars außer der wenigstens einen elastischen Fläche (2) mit einem wärmeisolierenden Material (4) bedeckt ist.

4. Scharfer/stumpfer Trokar gemäß Anspruch 3, wobei das wärmeisolierende Material ein Aerogel umfasst.

5. Scharfer/stumpfer Trokar gemäß einem der Ansprüche 1 bis 4, weiterhin umfassend wenigstens einen Ausflusskanal (8), der über einen Dichtungsteil (10) dicht an wenigstens einen der Kanäle (1) angeschlossen ist, um Kühlmittel zu leiten, und an seinem distalen Ende eine Düse (9) aufweist, die so konfiguriert ist, dass sie die Effizienz und Zielführung der elastischen Fläche (2) erhöht.

## Revendications

1. Trocart aiguisé/émoussé avec une sonde cryogénique pour cryogéniser des tissus contenant des canaux étanches pour conduire le réfrigérant dans un flux continu ou interrompu algorithmique, la sonde cryogénique étant raccordée à une pompe et à un récipient pour le réfrigérant et se terminant par une tête cryogénique ayant au moins une surface élastique (2) configurée pour modifier sa forme et se distendre suite à l'écoulement du gaz pompé sous pression à travers le trocart afin de former une chambre de décompression (5), dans laquelle le réfrigérant est amené par le biais des canaux (1) et ensuite subit la décompression et l'évaporation provoquant le refroidissement de la surface élastique (2) de la tête cryogénique, dans lequel la au moins une surface élastique (2) est configurée pour s'adapter au tissu exposé à la cryogénisation (3) et entourer partiellement le tissu traité, et dans lequel la au moins une surface élastique (2) est agencée sur la paroi latérale de la tête cryogénique.

2. Trocart aiguisé/émoussé selon la revendication 1, dans lequel la au moins une surface élastique (2) comprend un matériau sélectionné parmi le PEBA, le graphène, le PET ou des polymères.

3. Trocart aiguisé/émoussé selon la revendication 1 ou 2, dans lequel la surface du trocart, excepté pour la au moins une surface élastique (2), est recouverte par un matériau thermiquement isolant (4).

4. Trocart aiguisé/émoussé selon la revendication 3, dans lequel le matériau thermiquement isolant comprend un aérogel.

5. Trocart aiguisé/émoussé selon l'une quelconque des revendications 1 à 4, comprenant en outre au moins un canal de sortie (8) scellé via la partie d'étanchéité (10) sur au moins l'un des canaux (1) pour amener le réfrigérant et ayant, au niveau de son extrémité distale, une buse (9) configurée pour augmenter l'efficacité et le ciblage de la surface élastique (2).
